(19) Europäisches Patentamt
European Patent Office
Office européen des brevets

(11) Publication number: 0 251 466 A2

# (12) EUROPEAN PATENT APPLICATION

(21) Application number: 87304405.1

(22) Date of filing: 18.05.87

(51) Int. Cl.4: C07C 69/753 , C07C 69/757 , C07C 67/317

The title of the invention has been amended (Guidelines for Examination in the EPO, A-III, 7.3).

(30) Priority: 23.06.86 GB 8615313

(43) Date of publication of application: 07.01.88 Bulletin 88/01

(84) Designated Contracting States: CH DE FR GB LI NL

(71) Applicant: IMPERIAL CHEMICAL INDUSTRIES PLC
Imperial Chemical House Millbank
London SW1P 3JF(GB)

(72) Inventor: Cleare, Peter John Vernon
29 Gainsborough Drive
Ascot Berkshire(GB)
Inventor: Saville-Stones, Elizabeth Anne
43 Bedges Road
Wokingham Berkshire(GB)
Inventor: Smith, Douglas John
104 Deep Field Road
Bracknell Berkshire(GB)

(74) Representative: Alner, Henry Giveen Hamilton et al
Imperial Chemical Industries PLC Legal Department: Patents P.O. Box 6 Bessemer Road
Welwyn Garden City Herts, AL7 1HD(GB)

## (54) Chemical process for the preparation of esters of substituted cyclopropyl caboxylic acids.

(57) A process for the preparation of a compound of the general formula (III):

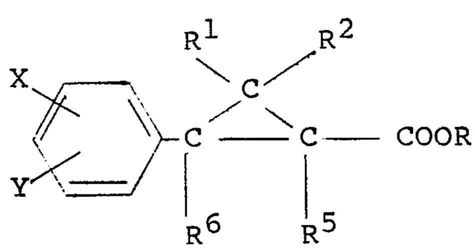

(III)

wherein $R^1$, $R^2$, $R^5$ and $R^6$ each represent a hydrogen atom or an alkyl group containing from I to 4 carbon atoms, R is an alkyl group and X and Y each represent a hydrogen atom, a halogen atom or an alkyl, cycloalkyl, alkenyl, alkynyl, aryl, aralkyl, alkoxy or aryloxy group, the process comprising the elimination of the molecule HZ, with a simultaneous ring closure, from a compound of the general formula (IV):

EP 0 251 466 A2

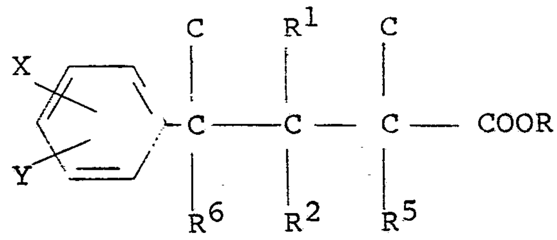

(IV)

wherein $R^1$, $R^2$, $R^5$ and $R^6$ R, X and Y have the same meanings as before and Z is a leaving group, for example a chlorine or bromine atom or a tosylate or mesylate group.

## CHEMICAL PROCESS

This invention relates to a process for the preparation of certain cyclopropane carboxylic esters which are valuable as intermediates in the manufacture of cyclopropane ring-containing tertiary amines having fungicidal activity.

In our British Specification No. 8501169 we have described compounds of the general formula (I) :

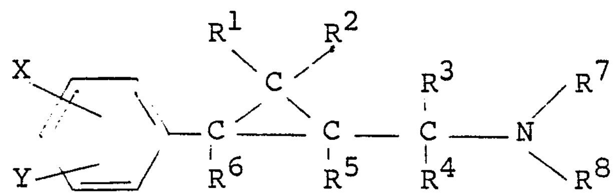

(I)

wherein $R^1$, $R^2$, $R^3$, $R^4$, $R^5$ and $R^6$ each represent a hydrogen atom, an alkyl group containing 1 to 4 carbon atoms or a halogen atom, $R^7$ and $R^8$ each represent a hydrogen atom or an alkyl group containing 1 to 4 carbon atoms or together form a ring which may contain an additional hetero atom, X and Y each represent a hydrogen or a halogen atom, or an alkyl, cycloalkyl, alkenyl, alkynyl, aryl, aralkyl, alkoxy or aryloxy group, or a

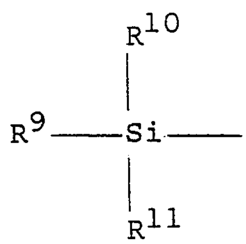

group wherein $R^9$, $R^{10}$, $R^{11}$ can be alkyl, alkenyl, alkynyl, cycloalkyl, or aryl. These compounds, including the stereoisomers thereof, and their addition salts, are valuable as fungicides. In particular, a high degree of fungicidal activity is observed in those compounds in which the two molecular residues

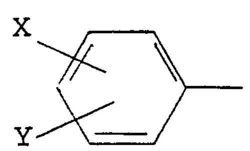

and

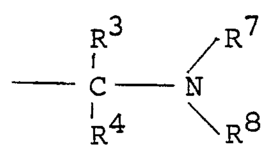

are attached to the cyclopropane ring in the trans position relative to one another.

In the above-mentioned specification, we also describe processes for producing the compounds of general formula (I). These processes involve a number of successive reaction steps, including the preparation as one of the intermediates of the carboxylic acid having the general formula (II) :

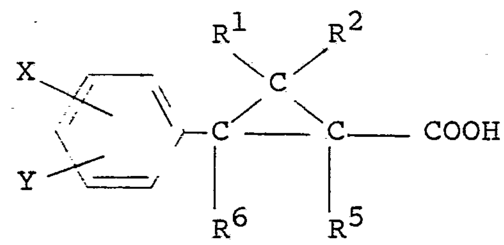

(II)

wherein $R^1$, $R^2$, $R^5$, $R^6$ X and Y have their previous meanings, or of a reactive derivative of this acid such as the acid chloride or an ester. In the processes in question, however, we have not up to the present time succeeded in carrying out any of the reaction steps with a marked degree of stereospecificity, so that the final product (I) is usually obtained as a mixture of stereoisomers, from which the particularly desired trans isomer has to be separated in a further operation.

We have now devised a process for the preparation of esters of certain of the cyclopropane carboxylic acids of formula (II) which is highly stereospecific, yielding a product which consists overwhelmingly of the trans isomer and which can be converted to the corresponding trans isomer of the tertiary amine (I), thus avoiding the need for a separation step.

Thus according to the present invention there is provided a process for the preparation of a compound of the general formula (III) :

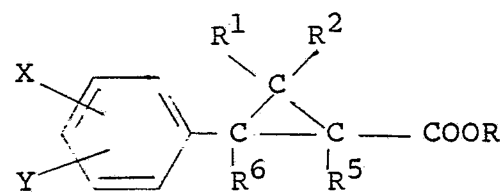

(III)

wherein $R^1$, $R^2$, $R^5$ and $R^6$ each represent a hydrogen atom or an alkyl group containing from I to 4 carbon atoms, R is an alkyl group and X and Y each represent a hydrogen atom, a halogen atom or an alkyl, cycloalkyl, alkenyl, alkynyl, aryl, aralkyl, alkoxy or aryloxy group, the process comprising the elimination of the molecule HZ, with simultaneous ring closure, from a compound of the general formula (IV) :

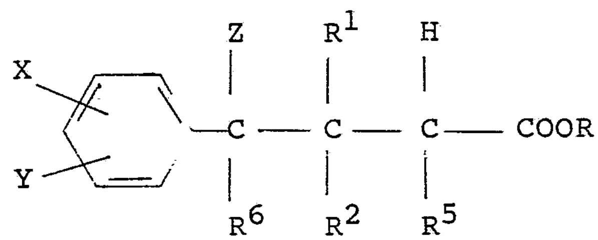

(IV)

wherein $R^1$, $R^2$, $R^5$, $R^6$ R, X and Y have the same meanings as before and Z is a leaving group, for example a chlorine or bromine atom or a tosylate or mesylate group.

The elimination and ring closure reaction of the compound (IV) may be effected in known manner, e.g., in the case where the leaving group Z is any of those just mentioned, by the action of sodium ethoxide in ethanol. The stereospecificity of these reactions is such that they result overwhelmingly, e.g. to the extent of about 90% or more, in the formation of the trans isomer of the compound (III) and only to a minor extent in the formation of the cis isomer.

The compound (IV) may be obtained in various ways depending upon its particular structure. For example, in the case where Z is a chlorine atom and $R^6$ is a hydrogen atom, this chloro ester may readily be obtained by one of the following procedures from a keto acid of the general formula (V) :

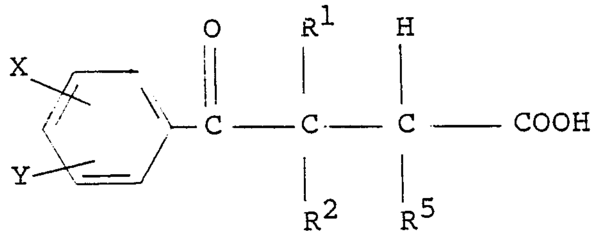

(V)

In the first procedure, the acid (V) is first converted to the alkyl ester (VI) in conventional manner :

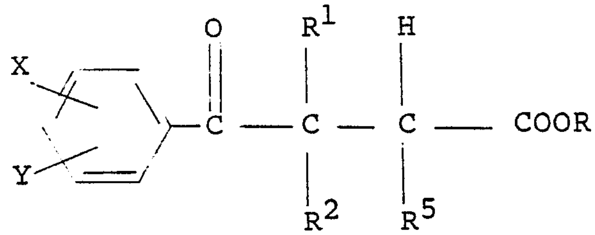

(VI)

and the ester is then reduced, e.g. with sodium borohydride in aqueous alkaline solution. The product of this reduction is a mixture of the hydroxy ester (VII) and the corresponding lactone (VIII) :

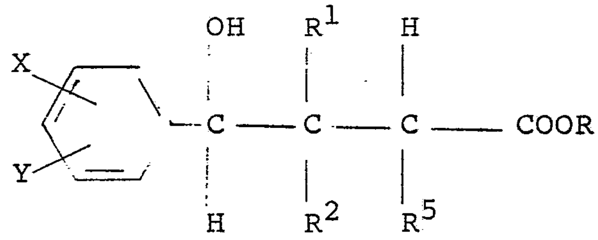

(VII)

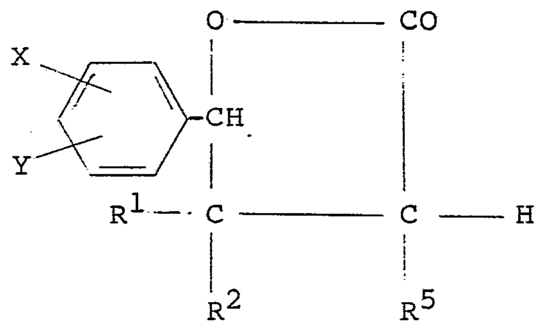

(VIII)

This mixture, without previous separation, is treated with thionyl chloride and then with the alcohol ROH to yield the desired chloro ester (IV).

In an alternative procedure for making the chloro ester, the keto acid (V) is reduced directly with sodium borohydride in aqueous alkaline solution, giving a mixture of the free hydroxy acid corresponding to the ester (VII) above and the lactone (VIII). This mixture is then treated successively with thionyl chloride and with the alcohol ROH to yield the chloro ester (IV).

The keto acid of formula (V) may readily be made by performing a Friedel-Crafts reaction upon a mixture of the aromatic hydrocarbon (IX) and the substituted succinic anhydride (X) :

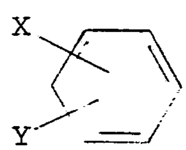

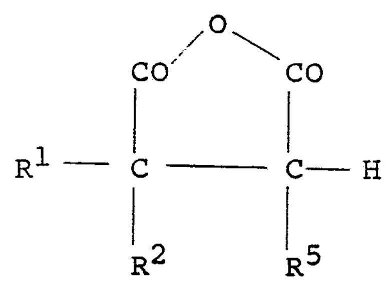

(IX) (X)

In the case where $R^6$ in the compound (IV) is an alkyl group containing from I to 4 carbon atoms, the compound in question may for example be obtained from the keto-acid (V) or the corresponding ester (VI) by treatment with a Grignard reagent $R^6MgHal$ or an organolithium reagent $R^6Li$., giving the tertiary alcohol (XI) and/or the lactone (XII):

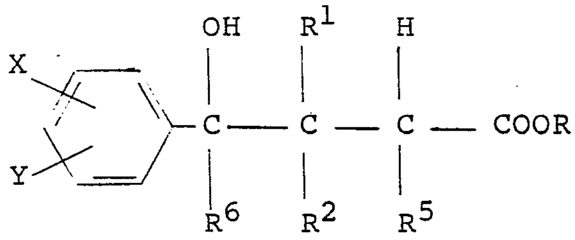

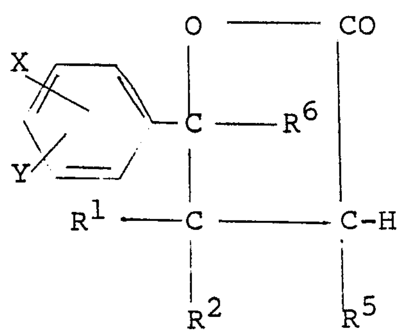

(XI)

(XII)

This compound or mixture, without previous separation, is treated with thionyl chloride and then with the alcohol ROH to yield the desired chloro-ester (IV).

The process of the present invention is of special interest for the preparation of the trans isomers of the esters falling within the general formula (III) but having the particular formula (XIII) :

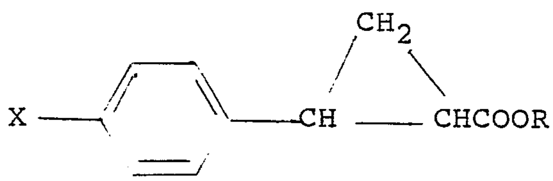

(XIII)

wherein X is an alkyl group containing 4 or 5 carbon atoms or a cyclohexyl group. In the case where X is a tertbutyl group, for example, the required starting material (IX) and (X) are tert-butylbenzene and succinic anhydride, and the final product is a mixture of the cis and trans isomers of the compound (XIII) of which the trans isomer constitutes about 90%.

The products (III) of the process of the invention are, as previously indicated, of value as intermediates in the manufacture of the fungicidally active compounds (I). A typical reaction sequence for converting a compound (III) into a compound (I) is as follows. The cyclopropane carboxylic ester (III) is first of all hydrolysed to the free carboxylic acid, and the latter is then converted to the acid chloride. The acid chloride is reacted with a primary or secondary amine of the formula $NHR^7R^8$ (see formula (I)), and finally the carbonyl group in the resulting amide is reduced to a methylene group. All of these reaction steps may be carried out by known standard methods. Thus, for example, the trans isomer of the compound (XIII), where R is an ethyl group, viz. ethyl 2-(p-tert-butylphenyl)-cyclopropane carboxylate, may be hydrolysed to the free acid by treatment with aqueous alcoholic potassium hydroxide, the acid isolated and then converted to the acid chloride by means of thionyl chloride in chloroform, and the acid chloride reacted with 2,6-dimethylmorpholine. The resulting 4-[2-(p-tert-butylphenyl)-cyclopropane carbonyl]-2,6-dimethylmorpholine is finally reduced with lithium aluminium hydride in ether or TMF to yield the tertiary amine 4-[2-(p-tert-butylphenyl)-trans-cyclopropylmethyl]-2,6-dimethylmorpholine.

The stereospecificity of the process of the invention resulting in the production of the cyclopropane carboxylic esters III in overwhelmingly the trans form is preserved during the subsequent conversion of the ester to the tertiary amine, so that the latter is obtained almost wholly as the highly fungicidally active trans isomer. There is normally no need to separate this from the relatively small amount present of the fungicidally less active cis isomer, in contrast to the processes described in our Specification No. 8501169 which result in more substantial proportions being present of the cis isomer and which therefore necessitate a final separation step.

The invention is illustrated by the following examples, in which temperatures are expressed in degrees Centigrade. Examples 1 to 13 describe the preparation of carboxylic acids and esters and these can be converted to the fungicidal amines as described in Example 4.

EXAMPLE I

A. Preparation of 4-(p-t-butylphenyl)-4-oxo-butanoic acid

Aluminium chloride (267g, 2.0mol) was stirred with dichloromethane (500ml) at room temperature and succinic anhydride (I00g, I.0mol) was added. The resulting suspension was heated at reflux for I.5 hours and recooled to room temperature. t-Butylbenzene (I74g, I.3mol) was then added over a period of 40 minutes with consequent evolution of hydrogen chloride gas. After the addition the mixture was stirred for a further I0 minutes before heating to reflux for I5 minutes and again cooling to room temperature. 2M Hydrochloric acid (2l) was introduced slowly and carefully with vigorous stirring. The phases were separated and the aqueous phase was extracted with dichloromethane (500ml). The combined organic phases were washed with water (2 × 500ml) and concentrated to a red semi-solid residue. This was recrystallised from cyclohexane/industrial methylated spirits (95/5 v/v) (7ml/g) to give the title compound (I26g) as a pale yellow solid mp II9-2I° (cf. I23.5-I25° ref. J.Org.Chem. I954, I9, 803; I26° ref. J.Org.Chem. I942, 7 , 5I7).

B. Preparation of ethyl 4(p-t-butylphenyl)-4-oxobutanoate

4-(p-t-Butylphenyl)-4-oxobutanoic acid (234g, Imol) was suspended in ethanol (750ml). Conc. sulphuric acid (5ml) was added and the mixture was heated at reflux for 5.5 hours. The resulting solution was cooled to room temperature and discharged into water (I.5l). This was extracted with diethyl ether (2 × 500ml). The combined extracts were washed with water (500ml), I% aqueous sodium hydroxide (2 × 250ml) and again with water (2 × 500ml) and dried over anhydrous magnesium sulphate. Removal of the solvent under reduced pressure gave the title compound (236g) as an oil bp.

C. Preparation of ethyl 2-(p-t-butylphenyl)cyclopropanecarboxylate, (method I)

A solution of sodium borohydride (8.55g, 0.225mol) was prepared in water (50ml) to which had been added 30% aqueous sodium hydroxide (I.0ml). Ethyl 4-(p-t-butylphenyl)-4-oxobutanoate (I98g, 0.75mol) was dissolved in ethanol (500ml). The sodium borohydride solution was then added dropwise over a period of I0 minutes whilst the temperature was maintained at below 40° by water cooling. Stirring was continued at room temperature for ca. 3 hours after which water (500ml) was added. The mixture was acidified to pHI by cautious addition of IM hydrochloric acid and toluene (500ml) was added. The phases were stirred together and then separated and the organic phase was washed with water (500ml) and concentrated under reduced pressure. A residual straw coloured oil (I84g) was obtained comprising a crude mixture of ethyl 4-(p-t-butylphenyl)-4-hydroxybutanoate and 4-(p-t-butylphenyl)-4-butyrolactone.

The crude oil (I84g) prepared as described above was dissolved in dichloromethane (500ml) and cooled to I0°C. Thionyl chloride (200ml) was added over a period of I5 minutes during which time the temperature increased to I7°C and hydrogen chloride and sulphur dioxide gases were evolved. Stirring was continued for a further 2 hours. Ethanol (200ml) was then added cautiously over I hour at I0-I7°C causing vigorous gas evolution. The solution was stirred at room temperature overnight before concentrating under reduced pressure to obtain a yellow mobile oil (270g) which was used directly in the next-stage.

Sodium (42g, I.83mol) was dissolved portionwise in ethanol (Il) over I hour whilst the temperature increased to 70°C. The solution was allowed to cool to 50°C, the oil (250g), prepared as described immediately above, was added and the mixture was heated at reflux for 2 hours. After cooling to room temperature the mixture was poured into water (2l), acidified with conc. hydrochloric acid, and extracted with toluene (2 × 500ml). The combined extracts were washed with water (2 × 500ml) and the solvent was removed under reduced pressure to leave a dark oil (I88g). This was distilled via a column (I5cm length, packed with Knitmesh) to obtain ethyl 2-(p-t-butylphenyl)-cis /trans-cyclopropanecarboxylate as a colourless oil. Two fractions were collected boiling within the range 96I02°/0.Imm: Fraction I,85g ( > 90% as trans isomer) and Fraction 2,50g (>75% as trans isomer).

D. Preparation of ethyl 2-(p-t-butylphenyl)-cyclopropanecarboxylate, (method 2)

A solution of sodium borohydride (6.5g, 0.17mol) was prepared in water (50ml). 1M Aqueous sodium hydroxide (170ml) was diluted with water (150ml) and 4-(p-t-butylphenyl)-4-oxobutanoic acid (40g, 0.17mol) was added to dissolve. This solution was cooled to ca. 0°C and the sodium borohydride solution was then added over a period of 20 minutes with brisk stirring. The resulting thick white suspension was stirred at room temperature for a further 2 hours and then poured cautiously into 2M hydrochloric acid (200ml). The mixture was extracted with dichloromethane (3 $\times$ 100ml) and the combined extracts were washed with water (2 $\times$ 200ml) and dried over anhydrous magnesium sulphate. Removal of the solvent under reduced pressure afforded a white solid (37.5g) which comprised a crude mixture of 4-(p-t-butylphenyl)-4-hydroxybutanoic acid and 4-(p-t-butylphenyl)-4-butyrolactone.

The above described solid product (4.36g) was dissolved in ethanol (10ml) and thionyl chloride (11.7ml, 0.16mol) was added dropwise over 10 minutes whilst the temperature was held at ca. 30°C by external cooling. When the gas evolution had subsided more ethanol (0.5ml) was introduced followed by more thionyl chloride (1.0ml). This procedure was repeated for a further four times and the mixture was then heated to reflux. After cooling the solution was concentrated under reduced pressure to obtain a dark orange oil (5.42g) which was used directly for the next stage.

Sodium (1.75g, 0.076mol) was dissolved portionwise in ethanol (20ml). The oil (5.42g), prepared as described immediately above, was dissolved in toluene and added slowly to the sodium ethoxide solution at room temperature. The mixture was allowed to stand overnight at room temperature and then heated at 40°C for 1 hour. After recooling it was poured into iced water (100ml), acidified with dilute hydrochloric acid, and extracted with diethyl ether (3 $\times$ 50ml). The combined extracts were washed with water (2 $\times$ 100ml), dried over anhydrous magnesium sulphate and the solvent was removed under reduced pressure to leave a dark red liquid (3.89g). This was distilled at ca. 0.2mm pressure using a Kugelröhr apparatus (oven temperature ca. 140°C) to obtain ethyl 2-(p-t-butyl-phenyl)-cis/trans-cyclopropanecarboxylate as a pale yellow liquid (1.95g) (ca. 90% as trans isomer).

EXAMPLE 2

A. Preparation of 4-(p-n-butylphenyl)-4-oxo-butanoic acid

Succinic anhydride (20g, 0.2mol) was stirred in n-butylbenzene (67g, 0.5mol) and powdered aluminium chloride (53.3g, 0.4mol) added portionwise over a period of 15 minutes. After the addition the resulting suspension was heated at 80°C for 10 hours and cooled to room temperature. The dark red oil was poured carefully into ice-water and extracted with ethyl acetate (3 $\times$ 150ml). The ethyl acetate extracts were washed with saturated sodium bicarbonate (3 $\times$ 150ml) and the aqueous extract acidified with concentrated hydrochloric acid. Extraction of the aqueous extract with ethyl acetate followed by drying over anhydrous sodium sulphate and removal of the solvent in vacuo gave a light brown solid. Washing this solid with ice cold petroleum ether gave the title compound (24.7g, 53%) as a white crystalline solid m.p. 135-136°C.

B. Preparation of ethyl 4-(p-n-butylphenyl)-4-oxobutanoate

4-(p-n-Butylphenyl)-4-oxobutanoic acid (9.10g, 0.039mol) was dissolved in ethanol (150ml) and hydrogen chloride gas was bubbled through the solution for about 1 hour. After saturating the solution it was refluxed under reduced pressure. The residue was dissolved in diethyl ether, washed with water and dried over anhydrous magnesium sulphate. Removal of the solvent under reduced pressure gave the title compound (10.15g, 100%) as a pale yellow oil.

C. Preparation of trans ethyl 2-(p-n-butylphenyl)-cyclopropane carboxylate

A solution of sodium borohydride (0.5g, 0.013mol) was prepared in water (3ml) to which had been added 30% aqueous sodium hydroxide (2 drops). Ethyl 4-(p-n-butylphenyl)-4-oxobutanoate (9.36g, 0.036mol) was dissolved in ethanol (25ml) and the sodium borohydride solution was then added dropwise over a period of 10 minutes whilst the temperature was maintained at below 40°C by water cooling. Stirring was continued at room temperature for 3 hours after which water (25ml) was added. The mixture was acidified to pH 1 by

cautious addition of IM hydrochloric acid and extracted with diethyl ether (2 × 100ml). The ethereal extracts were washed with water, dried over anhydrous magnesium sulphate and concentrated under reduced pressure. A residual straw coloured oil (8.5g) was obtained comprising of a crude mixture of 4-(p-n-butylphenyl)-4-hydroxybutanoate and 4-(p-n-butylphenyl)-4-butyrolactone.

The crude oil (8.4g) prepared as described above was dissolved is dichloromethane (45ml) and cooled to 10°C. Thionyl chloride (15ml) was added over a period of 15 minutes and stirring was continued for a further 2 hours. Ethanol (25ml) was then added cautiously over I hour at 10-20°C causing vigorous gas evolution. The solution was stirred at room temperature overnight before concentrating under reduced pressure to obtain a yellow oil (6.5g) which was used directly in the next stage.

Metallic sodium (0.92g, 0.04mol) was dissolved in ethanol (20ml) over I hour and the oil (5.6g, 0.02mol), prepared as described immediately above, was added at 50°C. The mixture was heated at reflux for 2 hours, cooled to room temperature, poured into water, acidified with concentrated hydrochloric acid and extracted with diethyl ether (2 × 50ml). The ethereal extracts were washed with water, dried over anhydrous magnesium sulphate and the solvent removed <u>in vacuo</u> to give an orange oil. This was purified by column chromatography (silica gel eluted with hexane/ethyl acetate 9:1) to give the title compound (3.5g) as the <u>trans</u> isomer.

EXAMPLE 3

A. <u>Preparation of 4-( -cyclohexylphenyl)-4-oxo-butanoic acid</u>

Succinic anhydride (20g, 0.2mol) was stirred in cyclohexylbenzene (80.1g, 0.5mol) and powdered aluminium chloride (53.3g, 0.4mol) added portionwise over a period of 15 minutes. After the addition the resulting suspension was heated at 80°C for 12 hours and cooled to room temperature. The viscous dark red oil was poured carefully into ice-water and extracted with ethyl acetate (3 × 150ml). The ethyl acetate extracts were washed with saturated sodium bicarbonate (3 × 150ml) and the aqueous extract acidified with concentrated hydrochloric acid. Extraction of the aqueous extract with ethyl acetate followed by drying over anhydrous sodium sulphate and removal of the solvent <u>in vacuo</u> gave a light brown solid. Washing this solid with cold petroleum ether gave the title compound (12.4g, 24%) m.p. 125-126°C.

B. <u>Preparation of ethyl 4-(p-cyclohexylphenyl)-4-oxobutanoate</u>

4-(p-Cyclohexylphenyl)-4-oxo-butanoic acid (7.0g, 0.027mol) was dissolved in ethanol (100ml) and hydrogen chloride gas was bubbled through the solution for about I hour. After saturating the solution it was refluxed for I hour and the ethanol removed under reduced pressure. The residue was dissolved in diethyl ether, washed with water and dried over anhydrous magnesium sulphate. Removal of the solvent under reduced pressure gave the title compound (7.7g, 100%) as a pale yellow oil.

C. <u>Preparation of trans ethyl 2-(p-cyclohexylphenyl)cyclopropane carboxylate</u>

A solution of sodium borohydride (0.5g, 0.013mol) was prepared in water (3ml) to which had been added 30% aqueous sodium hydroxide (2 drops). Ethyl 4-(p-cyclohexylphenyl)-4-oxobutanoate (7.0g, 0.025mol) was dissolved in ethanol (25ml) and the sodium borohydride solution was then added dropwise over a period of 10 minutes whilst the temperature was maintained at below 40°C by water cooling. Stirring was continued at room temperature for 3 hours after which water (25ml) was added. The mixture was acidified to pH I by cautious addition of IM hydrochloric acid and extracted with diethyl ether (2 × 100ml). The ethereal extracts were washed with water, dried over anhydrous magnesium sulphate and concentrated under reduced pressure. A residual straw coloured oil (6.5g) was obtained comprising of a crude mixture of 4-(p-cyclohexylphenyl)-4-hydroxybutanoate and 4-(p-cyclohexylphenyl)-4-butyrolactone. The crude oil (6.5g) prepared as described above was dissolved in dichloro-methane (40ml) and cooled to 10°C. Thionyl chloride (15ml) was added over a period of 15 minutes and stirring was continued for a further 2 hours. Ethanol (25ml) was then added cautiously over I hour at 10-20C causing vigorous gas evolution. The solution was stirred at room temperature overnight before concentrating under reduced pressure to obtain a yellow oil (6.0g) which was used directly in the next stage.

Metallic sodium (0.92g, 0.04mol) was dissolved in ethanol (20ml) over I hour and the oil (6.0g, 0.0Ig), prepared as described immediately above, was added at 50°C. The mixture was heated at reflux for 2 hours, cooled to room temperature, poured into water, acidified with concentrated hydrochloric acid and extracted with diethyl ether (2 × 50ml). The ethereal extracts were washed with water, dried over anhydrous magnesium sulphate and the solvent removed in vacuo to give a yellow oil. This was purified by column chromatography (silica gel eluted with hexane/ethyl acetate 9:I) to give the title compound (4.0g) as the trans isomer.

EXAMPLE 4

Preparation of 4-[2-(p-t-butylphenyl)-cyclopropanecarbonyl]-2,6-dimethylmorpholine

Ethyl 2-(p-t-butylphenyl)-cis/trans-cyclopropanecarboxylate (73.8g, 0.3mol, >90% as trans isomer), prepared as described in Example IC was dissolved in methanol (400ml). A solution of potassium hydroxide (33.6g, ca 0.6mol) in water (400ml) was added and the mixture was heated at reflux for 5 hours. After cooling to room temperature the clear solution was poured into water (500ml) and extracted with dichloromethane (200ml). The aqueous phase was acidified to pHI with 5M hydrochloric acid whereupon an oil separated which was extracted into dichloromethane (2 × 200ml). The combined extracts were washed with water (2 × 200ml), dried over anhydrous magnesium sulphate and concentrated under reduced pressure to give 2-(p-t-butylphenyl)-cis/trans-cyclopropanecarboxylic acid (63g) as a crystalline solid m.p. 88-89°C.

The carboxylic acid (59.95g, 0.275mol) prepared as described immediately above was dissolved in chloroform (300ml) and N,N-dimethylformamide (2 drops) was added. Thionyl chloride (50ml) was introduced slowly and the mixture was stirred at room temperature and then heated at reflux for 2 hours by which time gas evolution had ceased. The solution was then allowed to cool to room temperature and concentrated under reduced pressure affording crude 2-(p-t-butylphenyl)-cis/trans-cyclopropanecarbonyl chloride as a colourless oil (65g).

A solution of 2,6-dimethylmorpholine (34.8g, 0.303mol, >95% as cis isomer) and pyridine (25ml, 0.3Imol) was prepared in dichloromethane (400ml). To this was added, over a period of I5 minutes, a solution of the above prepared acid chloride (65g) in dichloromethane (I00ml) whilst the temperature was maintained at<30°C. After stirring at room temperature for a further I.5 hours the mixture was poured into water (500ml). The phases were separated, the aqueous phase was re-extracted with dichloromethane (200ml) and the combined organic extracts then washed with 2N aqueous hydrochloric acid (2 × 200ml) and water (2 × 200ml) and dried over anhydrous magnesium sulphate. Removal of the solvent under reduced pressure gave the title compound as a pale red oil (85g).

EXAMPLE 5

Preparation of 4-[2-(p-t-butylphenyl)-trans-cyclopropylmethyl]-2,6-cis-dimethylmorpholine

Lithium aluminium hydride (2.2g, 0.058mol) was dispersed in tetrahydrofuran (40ml). To this was added, over a period of ca. I0 minutes, a solution of 4-[2-(p-t-butylphenyl)-cyclopropanecarbonyl]-2,6-dimethylmorpholine (2I.4g, 0.068mol) prepared as described above, in tetrahydrofuran (60ml) whilst the temperature was held at ca. 20°C by external cooling. After stirring overnight at room temperature ethyl acetate (ca. 50ml) was added carefully to destroy excess hydride reagents. The mixture was then poured into water (ca. I litre) and extracted with diethyl ether (2 × 50ml). The combined organic phases were washed with saturated brine (2 × 50ml) and dried over anhydrous magnesium sulphate. Finally the solvent was removed under reduced pressure to obtain a yellow oil (I6.8g) comprising the title compound (ca. 89%, w/w) together with its mixed isomers (ca. 4%, w/w).

**Claims**

I. A process for the preparation of a compound of the general formula (III) :

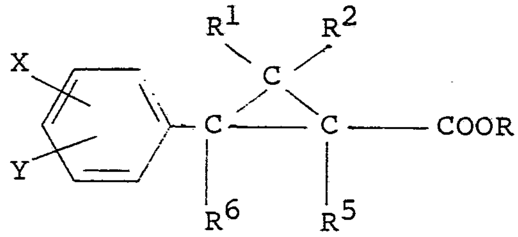

(III)

where $R^1$, $R^2$, $R^5$ and $R^6$ each represent a hydrogen atom or an alkyl group containing from I to 4 carbon atoms, R is an alkyl group and X and Y each represent a hydrogen atom, a halogen atom or an alkyl, cycloalkyl, alkenyl, alkynyl, aryl, aralkyl, alkoxy or aryloxy group, the process comprising the elimination of the molecule HZ, with simultaneous ring closure, from a compound of the general formula (IV) :

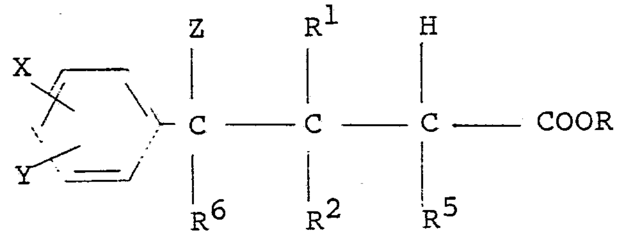

(IV)

wherein $R^1$, $R^2$, $R^5$ $R^6$ X and Y have the same meanings as before and Z is a leaving group.

2. A process as claimed in claim I wherein the reaction comprising elimination of the molecule HZ and ring closure is effected by the action of sodium ethoxide in ethanol.

3. A process as claimed in claim I or claim 2 wherein the compound (IV), in the case where Z is a chlorine atom and $R^6$ is a hydrogen atom, is obtained from a keto acid of the general formula (V):

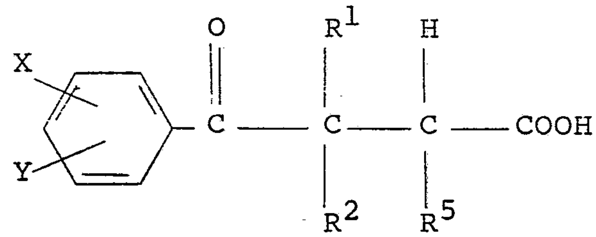

(V)

by either (a) converting the acid (V) to the alkyl ester (VI) in conventional manner:

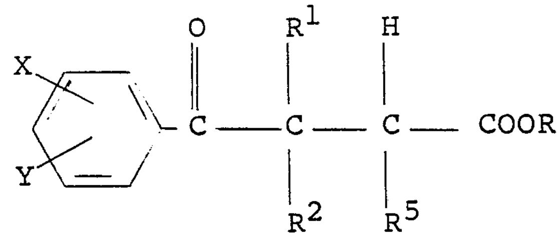

(VI)

and thereafter reducing the ester, for example with sodium borohydride in aqueous alkaline solution, to produce a mixture of the hydroxy ester (VII) and the corresponding lactone (VIII):

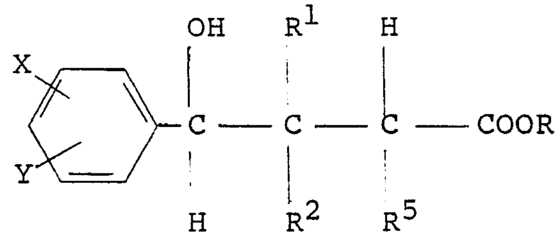

(VII)

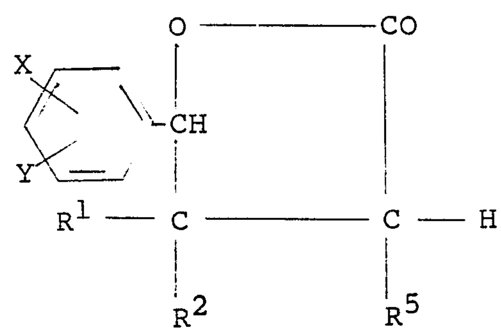

(VIII)

which mixture, without previous separation, is treated with thionyl chloride and then with the alcohol ROH to yield the desired chloro ester (IV); or (b) reducing the keto acid (V) directly with sodium borohydride in aqueous alkaline solution, giving a mixture of the free hydroxy acid corresponding to the ester (VII) and the lactone (VIII), which mixture is then treated successively with thionyl chloride and with the alcohol ROH to yield the chloro ester (IV); or (c) in the case where $R^6$ in the compound (IV) is an alkyl group containing from I to 4 carbon atoms by treatment of the ketoacid (V) or the corresponding ester (VI) with a Grignard reagent $R^6MgHal$ or an organolithium reagent $R^6Li$, giving the tertiary alcohol (XI) and/or the lactone (XII):

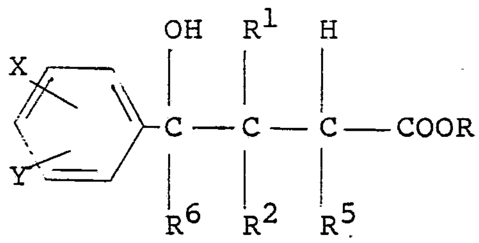

(XI)

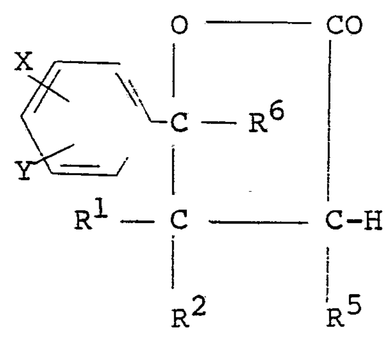

(XII)

which compound or mixture, without previous separation, is treated with thionyl chloride and then with the alcohol ROH to yield the desired chloro ester (IV).

4. A process as claimed in claim 3 wherein the keto acid of formula (V) is made by performing a Friedel-Crafts reaction upon a mixture of the aromatic hydrocarbon (IX) and the optionally substituted succinic anhydride (X):

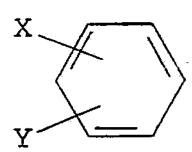

(IX)

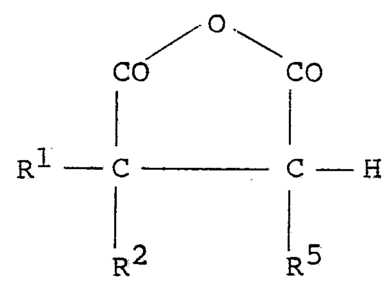

(X)

5. A process as claimed in any of the preceding claims for the preparation of <u>trans</u> isomers of the esters of formula:

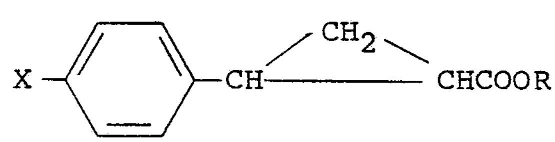

(XIII)

wherein X is an alkyl group containing 4 or 5 carbon atoms or a cyclohexyl group.

6. A process as claimed in claim 5 wherein X is a tertiary butyl group and the aromatic hydrocarbon (IX) is tertiary-butylbenzene and the anhydride (X) is succinic anhydride.

7. A process as claimed in any of the preceding claims and wherein the resultant products of formula (III) are hydrolysed to the free carboxylic acid which is converted to the acid chloride, the latter is then reacted with a primary or secondary amine of formula $NHR^7R^8$, and the carbonyl function in the resultant amide thereafter reduced to a methylene group, whereby there are produced compounds of general formula (I):

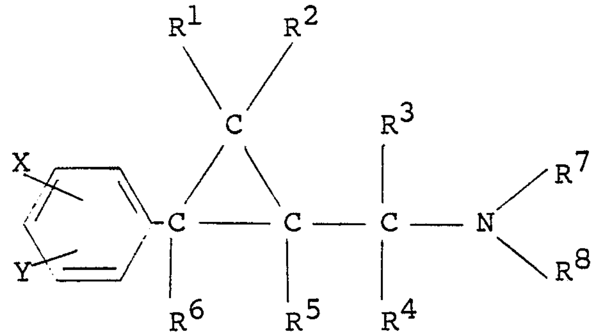

(I)

wherein $R^1$, $R^2$, $R^3$, $R^4$, $R^5$ and $R^6$ each represent a hydrogen atom, an alkyl group containing l to 4 carbon atoms or a halogen atom, $R^7$ and $R^8$ each represent a hydrogen atom or an alkyl group containing l to 4 carbon atoms or together form a ring which may contain an additional hetero atom, X and Y each represent a hydrogen or a halogen atom, or an alkyl, cycloalkyl, alkenyl, alkynyl, aryl, aralkyl, alkoxy or aryloxy group, or a

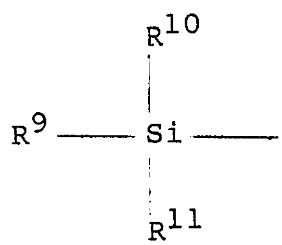

group wherein $R^9$, $R^{10}$ and $R^{11}$ can be alkyl, alkenyl, alkynyl, cycloalkyl or aryl.

8. A process for preparing the tertiary amine 4-[2-(p-tert-butylphenyl)-trans-cyclopropylmethyl]-2,6-dimethylmorpholine which comprises effecting the process as claimed in claim 6 to produce the trans isomer of the ester of formula (III) wherein R is ethyl, that is ethyl 2-(p-tert-butylphenyl)-cyclopropane carboxylate, hydrolysing this ester to the free acid by treatment with aqueous alcoholic potassium hydroxide, isolating the acid and then converting it to the acid chloride by means of thionyl chloride in chloroform, and reacting the acid chloride with 2,6-dimethylmorpholine to produce 4-[2-(p-tert-butylphenyl)-cyclopropane carbonyl]-2,6-dimethylmorpholine which is then reduced with lithium aluminium hydride in ether or TMF.

9. A process substantially as described in any of Examples l to 5, or any step, or combination of steps thereof.